# EUROPEAN PATENT APPLICATION

(11) **EP 3 984 553 A1**
(43) Date of publication of application: **20.04.2022**
(21) Application number: 20821775.2
(22) Date of filing: 12.06.2020
(51) Int. Cl.: A61K 39/395, A61K 31/7105, A61K 31/713, A61K 48/00, A61P 35/00

(54) **COMPOSITION FOR PREVENTING OR TREATING CANCER**

(30) Priority: 12.06.2019 KR 20190069334
(71) Applicant: Acurasysbio Co., Ltd., Seoul 06229 (KR)
(72) Inventor: LEE, Hyung Keun, Seoul 06279 (KR); JEON, Ji Won, Suwon-si Gyeonggi-do 16505 (KR); KIM, So Young, Suwon-si Gyeonggi-do 16357 (KR); YEO, A Reum, Seoul 06227 (KR)
(74) Representative: Schnappauf, Georg
(86) International application number: PCT/KR2020/007633
(87) International publication number: WO 2020/251297

(57) **Abstract**

The present invention relates to a composition capable of preventing or treating cancer, particularly pancreatic cancer. The composition can effectively inhibit the growth or proliferation of cancer cells, and furthermore, effectively prevent the resistance of cancer cells to anticancer agents, or the metastasis or recurrence of cancer, by using an inhibitor of the activity of at least one of interleukin-10 receptor subunit beta (IL-10RB, IL-10R2) and interleukin-22 (IL-22); or an inhibitor of expression of a gene encoding at least one of the interleukin-10 receptor subunit beta and the interleukin-22.

## Description

### Technical Field

The present invention is intended to provide a composition capable of preventing or treating cancer, particularly pancreatic cancer.

### Background Art

Cancer, also called tumor, is a cell mass composed of undifferentiated cells that proliferate unlimitedly irrespective of conditions required in tissues, unlike normal cells that can proliferate in a regular and controllable manner and can be suppressed, according to the individual's needs. These cancer cells that proliferate unlimitedly penetrate into surrounding tissues, and in more serious cases, metastasize to other organs of the body, resulting in an incurable disease that causes severe pain and eventually causes death.

Cancer is broadly classified into blood cancer and solid cancer, and occurs in almost all parts of the body, including pancreatic cancer, breast cancer, oral cancer, liver cancer, uterine cancer, esophageal cancer, and skin cancer. As therapeutic methods therefor, a small number of targeted therapeutic agents such as Gleevec or Herceptin have recently been used to treat certain cancers. However, up to now, surgery or radiotherapy, and anticancer therapy using chemotherapeutic agents that inhibit cell proliferation have been mainly used. However, existing chemotherapeutic agents are not targeted therapeutic agents. Thus, the biggest problems with the existing chemotherapeutic agents are side effects due to their cytotoxicity and drug resistance, which are the main factors that eventually result in failure of treatment despite initial successful response caused by the anticancer agents. Therefore, in order to overcome limitations of these chemotherapeutic agents, there is a continuous need for the development of targeted therapeutic agents with a clear anticancer mechanism of action.

Among these cancers, pancreatic cancer ranks seventh among the causes of cancer death worldwide, and has been reported to be the fifth leading cause of death in Korea. Pancreatic cancer is an aggressive disease compared to cancer diseases of other parts such as uterine cancer, breast cancer, rectal cancer, colorectal cancer, skin cancer, lung cancer, and liver cancer, but is very difficult to diagnose. In addition, pancreatic cancer is one of the most serious malignancies that develop acutely, are diagnosed lately, and show low survival rate. Thus, while the average survival rates of other types of cancer such as lung cancer increase, the average survival rate of pancreatic cancer tends to decrease because pancreatic cancer is difficult to diagnose and treat.

Compared with lung cancer which is the most typical cancer, pancreatic cancer not only has a fundamental difference that cancer develops in other organs, but also has a difference in terms of treatment due to different clinical pathological features and molecular biological features thereof. From a molecular biological point of view, it has been reported that mutations in the ras gene, an oncogene, appear in most (about 90%) of pancreatic cancers, which may lead to different therapeutic perspectives for pancreatic cancer, as compared to lung cancer or the like, for which mutations in the ras oncogene appear in a low proportion of 10%. In addition, most pancreatic cancer patients show a very aggressive course of progression and biological features compared to lung cancer patients, thereby being at risk of leading to a poor prognosis. Pancreatic cancer patients are locally advanced, have many difficulties to perform resection, usually show a metastatic disease, and often respond sensitively to side effects caused by intensive therapy. Only 10% to 15% of the patients are treatable with surgical resection. Even after fundamental treatment with surgery, a rate of recurrence remains very high.

Therefore, the development of anticancer agents that can treat pancreatic cancer in a more effective manner, and research on therapeutic strategies using the same have been continuously carried out. However, the actual clinical application of targeted therapeutic agents or immunotherapeutic agents, which have recently attracted attention, is limited to typical cancer diseases such as lung cancer, breast cancer, and gastric cancer. On the contrary, many cases where these agents do not exhibit the same effect even in pancreatic cancer have been reported. Accordingly, there is a growing demand for new therapeutic strategies against pancreatic cancer.

### DISCLOSURE

### Technical Problem

An object of the present invention is to provide a composition capable of preventing or treating cancer.

However, the objects to be achieved by the present invention are not limited to the object mentioned above, and other objects not mentioned herein will be clearly understood by those of ordinary skill in the art from the following description.

### Technical Solution

One embodiment of the present invention is directed to a pharmaceutical composition for preventing or treating cancer containing, as an active ingredient: an inhibitor of the activity of at least one of interleukin-10 receptor subunit beta (IL-10R2, IL-10RB) and interleukin-22 (IL-22); or an inhibitor of expression of a gene encoding at least one of the interleukin-10 receptor subunit beta and the interleukin-22.

The composition of the present invention may further contain an inhibitor of the activity of programmed death ligand 1 (PD-L1) or an inhibitor of expression of a gene encoding the programmed death-1, which may exhibit a synergistic effect on the prevention or treatment of cancer.

In the present invention, the "interleukin-10 receptor subunit beta (IL-10RB, IL-10R2)" belongs to the cytokine receptor family, and is an accessory chain essential for the active interleukin 10 receptor complex. Co-expression of the interleukin-10 receptor subunit beta and interroutine-10 receptor subunit alpha (IL10RA) protein is required for IL10-induced signal transduction. This gene and three other interferon receptor genes, IFNAR2, IFNAR1, and IFNGR2, form a class II cytokine receptor gene cluster located in a small region on chromosome 21. In the present invention, the interleukin-10 receptor subunit beta may consist of the amino acid sequence represented by SEQ ID NO: 1, but is not limited thereto. In addition, in the present invention, the interleukin-10 receptor subunit beta may be encoded by the nucleotide sequence represented by SEQ ID NO: 2, but is not limited thereto.

In the present invention, the "interleukin-22 (IL-22)" is an α-helical cytokine that binds to a heterodimeric cell surface receptor composed of IL-10R2 and IL-22R1 subunits. The interleukin-22 belongs to a group of cytokines called the IL-10 family or the IL-10 superfamily, and is classified as a potential regulator of cellular inflammatory responses. In the present invention, the interleukin-22 may consist of the amino acid sequence represented by SEQ ID NO: 3, but is not limited thereto. In addition, in the present invention, the interleukin-22 may be encoded by the nucleotide sequence represented by shown in SEQ ID NO: 4, but is not limited thereto.

In the present invention, the "programmed death ligand 1 (PD-L1)" is encoded by the CD274 gene, and is also called CD274 or B7 homolog 1 (B7-H1). The programmed death ligand 1 belongs to a 40-kDa type 1 transmembrane protein and plays a role in suppressing the adaptive arm of the immune system during particular events such as pregnancy, tissue allografts, autoimmune disease and other diseases such as hepatitis. Meanwhile, in the present invention, the programmed death ligand 1 may consist of the amino acid sequence represented by SEQ ID NO: 5, but is not limited thereto. In addition, in the present invention, the programmed death ligand 1 may be encoded by the nucleotide sequence represented by SEQ ID NO: 6, but is not limited thereto.

In the present invention, the interleukin-10 receptor subunit beta, interleukin-22 or programmed death ligand 1 may be present in peripheral blood mononuclear cells (PBMCs), preferably CD45⁺ cells, more preferably white blood cells. Accordingly, in the present invention, the inhibitor of the activity or the inhibitor of expression may inhibit the activity of the interleukin-10 receptor subunit beta, interleukin-22 or programmed death ligand 1 present in peripheral blood mononuclear cells (PBMCs), preferably CD45⁺ cells, more preferably white blood cells, or inhibit expression of the gene encoding the protein.

In the present invention, the inhibitor of the activity of the interleukin-10 receptor subunit beta, interleukin-22 or programmed death ligand 1 may include any one or more selected from the group consisting of compounds, peptides, peptide mimetics, aptamers, antibodies, and natural products, which bind specifically to these proteins.

In the present invention, the "peptide mimetics" is a peptide or non-peptide that inhibits a binding domain of the interleukin-10 receptor subunit beta, interleukin-22 or programmed death ligand 1 protein leading to the activity of the nterleukin-10 receptor subunit beta, interleukin-22 or programmed death ligand 1. Major residues of a nonhydrolyzable peptide analog may be produced using β-turn dipeptide cores, keto-methylene pseudopeptides, azepine, benzodiazepine, β-aminoalcohol, and a substituted gamma-lactam ring.

In the present invention, the "aptamer" is a single-stranded nucleic acid (DNA, RNA or modified nucleic acid) having a stable tertiary structure and capable of binding to a target molecule with high affinity and specificity. After an aptamer discovery technology called SELEX (Systematic Evolution of Ligands by EXponential enrichment) is first developed, many aptamers that can bind to various target molecules, including small molecules, peptides, and membrane proteins have been continuously discovered. The aptamer is comparable to a single antibody due to its ability to bind to a target molecule with unique high affinity (usually a pM level) and specificity, and particularly, has high potential as an alternative antibody to be referred to as a "chemical antibody".

In the present invention, the "antibody" may be either one produced by injection of the interleukin-10 receptor subunit beta, interleukin-22 or programmed death ligand 1, or a commercially available antibody. In addition, the antibodies include polyclonal antibodies, monoclonal antibodies, and fragments capable of binding to an epitope.

The polyclonal antibody may be produced by a conventional method of obtaining serum containing the antibody by injecting the interleukin-10 receptor subunit beta, interleukin-22 or programmed death ligand 1 into an animal and collecting blood from the corresponding animal. This polyclonal antibody may be purified by any method known in the art and obtained from any animal species host, such as goats, rabbits, sheep, monkeys, horses, pigs, cows, dogs, etc.

In addition, the monoclonal antibody may be produced using any technique that provides the production of antibody molecules through continuous culture of a cell line. Such techniques include, but are not limited to, hybridoma techniques, human B-cell line hybridoma techniques, and EBV-hybridoma techniques.

In addition, an antibody fragment containing a specific binding site for the interleukin-10 receptor subunit beta, interleukin-22 or programmed death ligand 1 may be produced. For example, a F(ab')₂ fragment may be produced by degrading an antibody molecule by pepsin, and a Fab fragment may be produced by reducing disulfide bridges of the F(ab')₂ fragment, but the production method is not limited thereto. Alternatively, monoclonal Fab fragments having the desired specificity may be identified quickly and easily by decreasing a Fab expression library.

In the present invention, the antibody may be bound to a solid substrate to facilitate subsequent steps such as washing or separation of the complex. Examples of the solid substrate include synthetic resins, nitrocellulose, a glass substrate, a metal substrate, glass fibers, microspheres, and microbeads. In addition, the synthetic resins include polyester, polyvinyl chloride, polystyrene, polypropylene, PVDF, and nylon.

In the present invention, the inhibitor of the activity may bind specifically to the epitope of interleukin-10 receptor subunit beta represented by SEQ ID NO: 7, and preferably, the pharmaceutical composition of the present invention may contain an antibody specific to the interleukin-10 receptor subunit beta, wherein the antibody may bind specifically to the epitope represented by SEQ ID NO: 7, but is not limited thereto.

In the present invention, the inhibitor of expression of the gene encoding the interleukin-10 receptor subunit beta, interleukin-22 or programmed death ligand 1 may include any one or more selected from the group consisting of an antisense nucleotide, small interfering RNA (siRNA), short hairpin RNA (shRNA), and ribozyme, which bind complementarily to the gene.

In the present invention, the "antisense nucleotide" interferes with the flow of genetic information from DNA to protein by binding (hybridization) to a complimentary nucleotide sequence of DNA, immature-mRNA or mature mRNA as defined by Watson-Crick base pairing. The specificity of antisense nucleotides to target sequences makes them exceptionally multifunctional. Since antisense-nucleotides are long chains of monomer units, they may be readily synthesized for the target RNA sequence. Many recent studies have demonstrated the utility of antisense nucleotides as biochemical means for studying target proteins. The use of antisense nucleotides may be considered as a novel form of inhibitor because of many recent advances in the field of oligonucleotide chemistry and synthesis of nucleotides that exhibit improved cell line adsorption, target binding affinity and nuclease resistance.

In the present invention, the "siRNA" and "shRNA" are nucleic acid molecules capable of mediating RNA interference or gene silencing. Since they may inhibit the expression of target genes, they are used as an efficient gene knockdown method or a gene therapy method. The shRNA has a hairpin structure formed by binding between complementary sequences within a single-stranded oligonucleotide. *In vivo*, the shRNA fragment may be cleaved by dicer into siRNA, which is a double-stranded oligonucleotide as a small RNA fragment having 21 to 25 nucleotides, and may bind specifically to mRNA having a sequence complementary thereto and inhibit expression thereof. Thus, any one of shRNA and siRNA for use may be selected by a person skilled in the art, and if the mRNA sequences targeted by them are the same, shRNA and siRNA may exhibit similar effects of reducing expression. For the purposes of the present invention, it may act specifically on the gene encoding the interleukin-10 receptor subunit beta, interleukin-22 or programmed death ligand 1 to cleave the interleukin-10 receptor subunit beta, interleukin-22 or programmed death ligand 1 gene (e.g., mRNA molecule), thereby inducing RNA interference (RNAi), thereby inhibiting the interleukin-10 receptor subunit beta, interleukin-22 or programmed death ligand 1. siRNA may be synthesized chemically or enzymatically. The method for producing siRNA is not particularly limited, and any method known in the art may be used. Examples of the method include, but are not limited to, a method of directly chemically synthesizing siRNA, a method of synthesizing siRNA using *in vitro* transcription, a method of enzymatically cleaving a long double-stranded RNA synthesized by *in vitro* transcription, an expression method using intracellular delivery of an shRNA expression plasmid or viral vector, and an expression method using intracellular delivery of a PCR (polymerase chain reaction)-induced siRNA expression cassette.

In the present invention, the "ribozyme" refers to an RNA molecule having catalytic activity. Ribozymes with various activities are known, and ribozymes for the interleukin-10 receptor subunit beta, interleukin-22 or programmed death ligand 1 gene include known or artificially produced ribozymes, Alternatively, ribozymes having target-specific RNA cleavage activity may be produced by known standard techniques.

In the present disclosure, the "cancer" refers to or describes the physiological condition in mammals that is typically characterized by unregulated cell growth. In the present invention, the cancer to be prevented or treated may be pancreatic cancer, thyroid cancer, breast cancer, biliary tract cancer, gallbladder cancer, colorectal cancer, uterine cancer, esophageal cancer, gastric cancer, brain cancer, rectal cancer, lung cancer, bladder cancer, kidney cancer, ovarian cancer, prostate cancer, head and neck cancer, skin cancer, blood cancer or liver cancer, depending on the site of occurrence. Preferably, the cancer may be pancreatic cancer.

In the present invention, administration of an inhibitor of the activity of at least one of interleukin-10 receptor subunit beta and interleukin-22; or an inhibitor of expression of a gene encoding at least one of the interleukin-10 receptor subunit beta and the interleukin-22 may effectively inhibit the growth or proliferation of cancer cells, preferably pancreatic cancer cells, and furthermore, may also effectively prevent or treat the resistance of cancer to drugs or the recurrence or metastasis of cancer. In addition, in the present invention, additional co-administration of an inhibitor of the activity of programmed death ligand 1 (PD-L1) or an inhibitor of the expression of a gene encoding the programmed death ligand 1 may provide a synergistic effect on the prevention or treatment of cancer.

In the present invention, "preventing" may include, without limitation, any action that blocks the occurrence of cancer symptoms or inhibits or delays the progression of cancer symptoms by using the pharmaceutical composition of the present invention.

In the present invention, "treating" may include, without limitation, any action that alleviates or beneficially changes cancer symptoms by using the pharmaceutical composition of the present invention.

The pharmaceutical composition of the present invention may also be administered in combination with other additional anticancer agents, thereby further enhancing the growth inhibitory effect on cancer cells.

Here, the anticancer agents may be one or more selected from the group consisting of nitrogen mustard, imatinib, oxaliplatin, rituximab, erlotinib, neratinib, lapatinib, gefitinib, vandetanib, nirotinib, semasanib, bosutinib, axitinib, cediranib, lestaurtinib, trastuzumab, gefitinib, bortezomib, sunitinib, carboplatin, bevacizumab, cisplatin, cetuximab, viscumalbum, asparaginase, tretinoin, hydroxycarbamide, dasatinib, estramustine, gemtuzumab ozogamicin, ibritumomab tiuxetan, heptaplatin, methylaminolevulinic acid, amsacrine, alemtuzumab, procarbazine, alprostadil, holmium nitrate chitosan, gemcitabine, doxyfluridine, pemetrexed, tegafur, capecitabine, gimeracin, oteracil, azacitidine, methotrexate, uracil, cytarabine, fluorouracil, fludagabine, enocitabine, flutamide, decitabine, mercaptopurine, thioguanine, cladribine, carmofur, raltitrexed, docetaxel, paclitaxel, irinotecan, belotecan, topotecan, vinorelbine, etoposide, vincristine, vinblastine, teniposide, doxorubicin, idarubicin, epirubicin, mitoxantrone, mitomycin, bleromycin, daunorubicin, dactinomycin, pyrarubicin, aclarubicin, pepromycin, temsirolimus, temozolomide, busulfan, ifosfamide, cyclophosphamide, melparan, altretmine, dacarbazine, thiotepa, nimustine, chlorambucil, mitolactol, leucovorin, tretonin, exmestane, aminoglutethimide, anagrelide, nabelbine, padrazole, tamoxifen, toremifene, testolactone, anastrozole, letrozole, vorozole, bicalutamide, lomustine, and carmustine, but is not limited thereto.

In the present invention, the pharmaceutical composition may be in the form of capsules, tablets, granules, injections, ointments, powders or beverages, and the pharmaceutical composition may be intended for humans.

For use, the pharmaceutical composition of the present invention may be formulated as oral dosage forms, including powders, granules, capsules, tablets and aqueous suspensions, preparations for external use, suppositories, and sterile injectable solutions, according to respective conventional methods, but is not limited thereto. The pharmaceutical composition of the present invention may contain pharmaceutically acceptable carriers. As the pharmaceutically acceptable carriers, binders, lubricants, disintegrants, excipients, solubilizing agents, dispersing agents, stabilizers, suspending agents, colorants, fragrances, and the like may be used for oral administration; buffers, preservatives, pain-relieving agents, solubilizing agents, isotonic agents, stabilizers, and the like may be used for injection; and bases, excipients, lubricants, preservatives, and the like may be used for local administration. The formulation of the pharmaceutical composition of the present invention may be prepared in various forms by mixing with the pharmaceutically acceptable carriers as described above. For example, for oral administration, the pharmaceutical composition of the present disclosure may be prepared in the form of a tablet, troche, capsule, elixir, suspension, syrup, wafer or the like, and for injection, the pharmaceutical composition may be prepared in the form of unit dosage ampoules or multiple dosage forms. In addition, the pharmaceutical composition may be prepared as solutions, suspensions, tablets, capsules, sustained-release formulations, or the like.

Meanwhile, examples of carriers, excipients and diluents, which are suitable for formulation, include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, mineral oil, and the like. In addition, the pharmaceutical composition may further contain fillers, anticoagulants, lubricants, wetting agents, fragrances, emulsifiers, preservatives, and the like.

Routes of administration of the pharmaceutical composition according to the present invention include, but are not limited to, oral, intravenous, intramuscular, intra-arterial, intra-marrow, intrathecal, intracardiac, transdermal, subcutaneous, intraperitoneal, intranasal, intestinal, local, sublingual and intrarectal routes. Oral or parenteral administration is preferred.

In the present invention, "parenteral" includes subcutaneous, intradermal, intravenous, intramuscular, intra-articular, intrabursal, intrasternal, intrathecal, intralesional and intracranial injection or infusion techniques. The pharmaceutical composition of the present invention may also be administered in the form of suppositories for rectal administration.

The dose of the pharmaceutical composition of the present invention may vary depending on various factors, including the activity of a particular compound used, the patient's age, body weight, general health, sex and diet, the time of administration, the route of administration, excretion rate, drug combination, and the severity of a particular disease to be prevented or treated. Although the dose of the pharmaceutical composition varies depending on the patient's condition and body weight, the severity of the disease, the form of drug, the route of administration, and the duration of administration, it may be appropriately selected by a person skilled in the art. The pharmaceutical composition may be administered at a dose of 0.0001 to 50 mg/kg/day or 0.001 to 50 mg/kg/day. The pharmaceutical composition of the present invention may be administered once or several times a day. The dose does not limit the scope of the present invention in any way. The pharmaceutical composition according to the present invention may be formulated as pills, sugar-coated tablets, capsules, liquids, gels, syrups, slurries, or suspensions.

Another embodiment of the present disclosure is directed to a method for preventing or treating cancer including a step of administering the pharmaceutical composition of the present invention to a subject in need of administration.

In the present invention, the "subject" is a subject suspected of having a cancer disease. The subject suspected of having the disease refers to mammals, including humans, rats, livestock, etc. that have or may develop the disease, but includes, without limitation, subjects that can be treated with the active ingredient provided by the present invention.

In the present invention, the cancer may be pancreatic cancer, thyroid cancer, breast cancer, biliary tract cancer, gallbladder cancer, colorectal cancer, uterine cancer, esophageal cancer, gastric cancer, brain cancer, rectal cancer, lung cancer, bladder cancer, kidney cancer, ovarian cancer, prostate cancer, head and neck cancer, skin cancer, blood cancer or liver cancer. Preferably, the cancer may be pancreatic cancer.

In the present invention, "administering" means providing the pharmaceutical composition of the present invention to a subject by any suitable method.

In the present invention, the formulation of the pharmaceutical composition that is administered as described above is not particularly limited, and may be administered as a solid formulation, a liquid formulation, or an aerosol formulation for inhalation. Specifically, the pharmaceutical composition may be administered as solid form preparations which are intended to be converted, shortly before use, to liquid form preparations for oral or parenteral administration. For example, the pharmaceutical composition may be formulated and administered as oral dosage forms, including powders, granules, capsules, tablets and aqueous suspensions, preparations for external use, suppositories, and sterile injectable solutions, but is not limited thereto.

In addition, in the present invention, pharmaceutically acceptable carriers may be additionally administered together with the pharmaceutical composition of the present invention. Here, as the pharmaceutically acceptable carriers, binders, lubricants, disintegrants, excipients, solubilizing agents, dispersing agents, stabilizers, suspending agents, colorants, fragrances, and the like may be used for oral administration; buffers, preservatives, pain-relieving agents, solubilizing agents, isotonic agents, stabilizers, and the like may be used for injection; and bases, excipients, lubricants, preservatives, and the like may be used for local administration. The formulation of the pharmaceutical composition of the present invention may be prepared in various forms by mixing with the pharmaceutically acceptable carriers as described above. For example, for oral administration, the pharmaceutical composition of the present disclosure may be prepared in the form of a tablet, troche, capsule, elixir, suspension, syrup, wafer or the like, and for injection, the pharmaceutical composition may be prepared in the form of unit dosage ampoules or multiple dosage forms. In addition, the pharmaceutical composition may be formulated as solutions, suspensions, tablets, capsules, sustained-release formulations, or the like.

Meanwhile, examples of carriers, excipients and diluents, which are suitable for formulation, include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, mineral oil, and the like. In addition, the pharmaceutical composition may further contain fillers, anticoagulants, lubricants, wetting agents, fragrances, emulsifiers, preservatives, and the like.

Routes of administration of the pharmaceutical composition according to the present invention include, but are not limited to, oral, intravenous, intramuscular, intra-arterial, intra-marrow, intrathecal, intracardiac, transdermal, subcutaneous, intraperitoneal, intranasal, intestinal, local, sublingual and intrarectal routes. Oral or parenteral administration is preferred.

In the present invention, "parenteral" includes subcutaneous, intradermal, intravenous, intramuscular, intra-articular, intrabursal, intrasternal, intrathecal, intralesional and intracranial injection or infusion techniques. The pharmaceutical composition of the present invention may also be administered in the form of suppositories for rectal administration.

In the present invention, "pharmaceutically effective amount" refers to a sufficient amount of an agent to provide a desired biological result. Said result may be reduction and/or alleviation of a sign, symptom, or cause of a disease, or any other desired alteration of a biological system. For example, an "effective amount" for therapeutic uses is the amount of the compound disclosed in the present invention, which is required to provide a clinically significant reduction in the disease. An appropriate "effective amount" in any individual case may be determined by one of ordinary skill in the art using routine experimentation. Thus, the expression "effective amount" generally refers to an amount in which an active substance has a therapeutic effect. In the case of the present invention, the active substance is an inhibitor of cancer cell growth, an agent for preventing, ameliorating or treating cancer, and an inhibitor of cancer metastasis.

The dose of the pharmaceutical composition of the present invention may vary depending on various factors, including the activity of a particular pharmaceutical composition used, the patient's age, body weight, general health, sex and diet, the time of administration, the route of administration, excretion rate, drug combination, and the severity of a particular disease to be prevented or treated. Although the dose of the compound varies depending on the patient's condition and body weight, the severity of the disease, the form of drug, the route of administration, and the duration of administration, it may be appropriately selected by a person skilled in the art. The pharmaceutical composition may be administered at a dose of 0.0001 to 100 mg/kg/day or 0.001 to 100 mg/kg/day. The pharmaceutical composition may be administered once or several times a day. The dose does not limit the scope of the present invention in any way. The pharmaceutical composition according to the present invention may be formulated as pills, sugar-coated tablets, capsules, liquids, gels, syrups, slurries, or suspensions.

The pharmaceutical composition of the present invention may be used alone or in combination with surgery, radiotherapy, hormone therapy, chemotherapy, and methods that use biological response modifiers.

Meanwhile, the method for preventing or treating cancer may be a combination therapy further including administering, for example, an anticancer agent as a substance having therapeutic activity against one or more diseases.

In the present invention, the "combination" should be understood to refer to simultaneous, individual or sequential administration. Where the administration is sequential or individual, the second component should be administered at intervals such that beneficial effects of the combination are not lost.

In the present invention, the anticancer agent may be one or more selected from the group consisting of nitrogen mustard, imatinib, oxaliplatin, rituximab, erlotinib, neratinib, lapatinib, gefitinib, vandetanib, nirotinib, semasanib, bosutinib, axitinib, cediranib, lestaurtinib, trastuzumab, gefitinib, bortezomib, sunitinib, carboplatin, bevacizumab, cisplatin, cetuximab, viscumalbum, asparaginase, tretinoin, hydroxycarbamide, dasatinib, estramustine, gemtuzumab ozogamicin, ibritumomab tiuxetan, heptaplatin, methylaminolevulinic acid, amsacrine, alemtuzumab, procarbazine, alprostadil, holmium nitrate chitosan, gemcitabine, doxyfluridine, pemetrexed, tegafur, capecitabine, gimeracin, oteracil, azacitidine, methotrexate, uracil, cytarabine, fluorouracil, fludagabine, enocitabine, flutamide, decitabine, mercaptopurine, thioguanine, cladribine, carmofur, raltitrexed, docetaxel, paclitaxel, irinotecan, belotecan, topotecan, vinorelbine, etoposide, vincristine, vinblastine, teniposide, doxorubicin, idarubicin, epirubicin, mitoxantrone, mitomycin, bleromycin, daunorubicin, dactinomycin, pyrarubicin, aclarubicin, pepromycin, temsirolimus, temozolomide, busulfan, ifosfamide, cyclophosphamide, melparan, altretmine, dacarbazine, thiotepa, nimustine, chlorambucil, mitolactol, leucovorin, tretonin, exmestane, aminoglutethimide, anagrelide, nabelbine, padrazole, tamoxifen, toremifene, testolactone, anastrozole, letrozole, vorozole, bicalutamide, lomustine, and carmustine, but is not limited thereto.

### Advantageous Effects

The pharmaceutical composition according to the present invention may effectively inhibit the growth or proliferation of cancer cells, and furthermore, may also effectively prevent or treat the resistance of cancer cells to anticancer agents, or metastasis or recurrence of cancer.

### Brief Description of Drawings

FIG. 1 is a graph showing the results of measuring the numbers of IL-10R2+CD45+ cells and IL-10R2-CD45+ cells in peripheral blood mononuclear cells derived from mice transplanted with pancreatic cancer cells in Experimental Example 1 of the present invention.
FIG. 2 is a graph showing the results of measuring the numbers of IL-10R2+CD45+ cells and IL-10R2-CD45+ cells in peripheral blood mononuclear cells derived from mice transplanted with pancreatic cancer cells after treatment of the mice with an anti-IL-10R2 antibody in Experimental Example 1 of the present invention.
FIG. 3 shows a photograph of pancreatic tumors in IL-22 gene knockout (K/O) mouse models transplanted with pancreatic cancer cells in Experimental Example 2 of the present invention.
FIG. 4 is a graph showing the results of measuring the weights of pancreatic tumors in IL-22 gene knockout (K/O) mouse models transplanted with pancreatic cancer cells in Experimental Example 2 of the present invention.
FIG. 5 is a graph showing the results of measuring the volumes of peripancreatic lymph nodes in IL-22 gene knockout (K/O) mouse models transplanted with pancreatic cancer cells in Experimental Example 2 of the present invention.
FIG. 6 is a photograph showing microscopic observation of pancreatic tumor tissue in IL-22 gene knockout (K/O) mouse models transplanted with pancreatic cancer cells in Experimental Example 2 of the present invention.
FIG. 7 is a graph showing the results of measuring the number of IL-10R2+7AAD- cells in PBMCs derived from IL-22 gene knockout (K/O) mice transplanted with pancreatic cancer cells in Experimental Example 3 of the present invention.
FIG. 8 is a graph showing the results of measuring the proportion of IL-10R2+CD11b+ cells in PBMCs derived from IL-22 gene knockout (K/O) mice transplanted with pancreatic cancer cells in Experimental Example 3 of the present invention.
FIG. 9 is a graph showing the results of measuring the number of IL-10R2+CD11b+7AAD- cells in PBMCs derived from IL-22 gene knockout (K/O) mice transplanted with pancreatic cancer cells in Experimental Example 3 of the present invention.
FIG. 10 depicts photographs showing microscopic observation after trichrome and Picrosirius red staining of the pancreatic tissues of IL-22 gene knockout (K/O) mice transplanted with pancreatic cancer cells in Experimental Example 4 of the present invention.
FIG. 11 is a graph showing the results of measuring the numbers of CD3+ cells, CD8+ cells and CD4+ cells separated from IL-22 gene knockout (K/O) mouse models transplanted with pancreatic cancer cells in Experimental Example 5 of the present invention.
FIG. 12 depicts graphs showing the results of measuring the absorbance after 24 hours, 48 hours or 72 hours of culture of pancreatic cancer cells in an IL-10RB+ or IL-10RB- conditioned medium supplemented with an anti-IL-10R2 antibody in Experimental Example 6 of the present invention.
FIG. 13 depicts graphs showing the results of measuring the number of cells after 48 hours of culture of pancreatic cancer cells in an IL-10RB+ or IL-10RB- conditioned medium supplemented with an anti-IL-10R2 antibody in Experimental Example 6 of the present invention.
FIG. 14 depicts graphs showing the results of measuring the number of cells after 72 hours of culture of pancreatic cancer cells in an IL-10RB+ or IL-10RB- conditioned medium supplemented with an anti-IL-10R2 antibody in Experimental Example 6 of the present invention.
FIG. 15 depicts graphs showing the results of measuring the number of cells after 48 hours or 72 hours of culture after adding IL-10R2+ cells or IL-10R2- cells to pancreatic cancer cells in Experimental Example 7 of the present invention.
FIG. 16 is a graph showing the results of measuring the number of cells after treating pancreatic cancer cells with anti-IL-10R2 antibody, anti-IL-22R1 antibody, anti-TNF-a antibody, anti-IFN-y antibody, anti-IL-2 antibody and anti-IL-6 antibody in Experimental Example 8 of the present invention.
FIG. 17 schematically shows an experimental design of Experimental Example 9 of the present invention.
FIG. 18 depicts photographs of pancreatic tissue after an anti-PD-Ll antibody and/or isotype antibody was administered to the IL-22 gene knockout (K/O) mouse models and wild-type (WT) mice transplanted with pancreatic cancer cells in Experimental Example 9 of the present invention.
FIG. 19 is a graph showing the results of measuring the weight of pancreatic tissue including cancer after an anti-PD-Ll antibody and/or isotype antibody was administered to the IL-22 gene knockout (K/O) mouse models and wild-type (WT) mice transplanted with pancreatic cancer cells in Experimental Example 9 of the present invention.
FIG. 20 depicts graphs showing the proportion of CD3+ CD8+ cells in immune cells that infiltrated into pancreatic tissue, measured after an anti-PD-L1 antibody and/or isotype antibody was administered to the IL-22 gene knockout (K/O) mouse models and wild-type (WT) mice transplanted with pancreatic cancer cells in Experimental Example 10 of the present invention.
FIG. 21 is a graph showing the number of CD3+ CD8+ cells in immune cells that infiltrated into pancreatic tissue, measured after an anti-PD-L1 antibody was administered to the IL-22 gene knockout (K/O) mouse models and wild-type (WT) mice transplanted with pancreatic cancer cells in Experimental Example 10 of the present invention.

### Best Mode

One embodiment of the present invention is directed to a pharmaceutical composition for preventing or treating cancer containing, as an active ingredient: an inhibitor of the activity of at least one of interleukin-10 receptor subunit beta (IL-10R2, IL-10RB) and interleukin-22 (IL-22); or an inhibitor of expression of a gene encoding at least one of the interleukin-10 receptor subunit beta and the interleukin-22.

The composition of the present invention may further contain an inhibitor of the activity of programmed death ligand 1 (PD-L1) or an inhibitor of expression of a gene encoding the programmed death ligand 1, which may exhibit a synergistic effect on the prevention or treatment of cancer.

In the present invention, the interleukin-10 receptor subunit beta, interleukin-22 or programmed death ligand 1 may be present in peripheral blood mononuclear cells (PBMCs), preferably CD45⁺ cells, more preferably white blood cells. Accordingly, in the present invention, the inhibitor of the activity or the inhibitor of expression may inhibit the activity of the interleukin-10 receptor subunit beta, interleukin-22 or programmed death ligand 1 present in peripheral blood mononuclear cells (PBMCs), preferably CD45⁺ cells, more preferably white blood cells, or inhibit expression of the gene encoding the protein.

In the present disclosure, the "cancer" may be pancreatic cancer, thyroid cancer, breast cancer, biliary tract cancer, gallbladder cancer, colorectal cancer, uterine cancer, esophageal cancer, gastric cancer, brain cancer, rectal cancer, lung cancer, bladder cancer, kidney cancer, ovarian cancer, prostate cancer, head and neck cancer, skin cancer, blood cancer or liver cancer. Preferably, the cancer may be pancreatic cancer.

### Mode for Invention

Hereinafter, the present invention will be described in more detail with reference to examples. It will be apparent to those of ordinary skill in the art that these examples serve merely to describe the present invention in more detail, and the scope of the present invention according to the subject matter of the present invention is not limited by these examples.

### Examples

### [Experimental Example 11

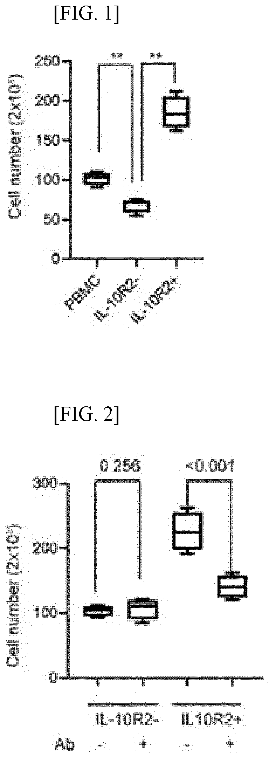

The pancreatic ductal adenocarcinoma cell line (Pan02) was injected directly into the pancreases of 8-week old wild-type (WT) mice (C57BL/6, OrientBio) at a concentration of 2 x 10⁶ cells/20 µL. After 14 days, peripheral blood mononuclear cells (PBMCs) were collected from the mice, and IL-10R2+CD45+ cells and IL-10R2-CD45+ cells were separated using a flow cytometer and the number of the cells was measured. The results of the measurement are shown in FIG. 1. In addition, after administration of an anti-IL-10R2 antibody (R&D Systems, Catalog number: MAB874) to the mice, PBMCs were collected, and IL-10R2+CD45+ cells and IL-10R2-CD45+ cells were separated using a flow cytometer and the number of the cells was measured. The results of the measurement are shown in FIG. 2.

As shown in FIG. 1, it could be confirmed that IL-10R2+CD45+ cells were highly expressed in the mice injected with the pancreatic cancer cells.

In addition, as shown in FIG. 2, as a result of administering the anti-IL-10R2 antibody to the mice injected with the pancreatic cancer cells, it could be confirmed that the number of IL-10R2+CD45+ cells significantly decreased.

### [Example 21

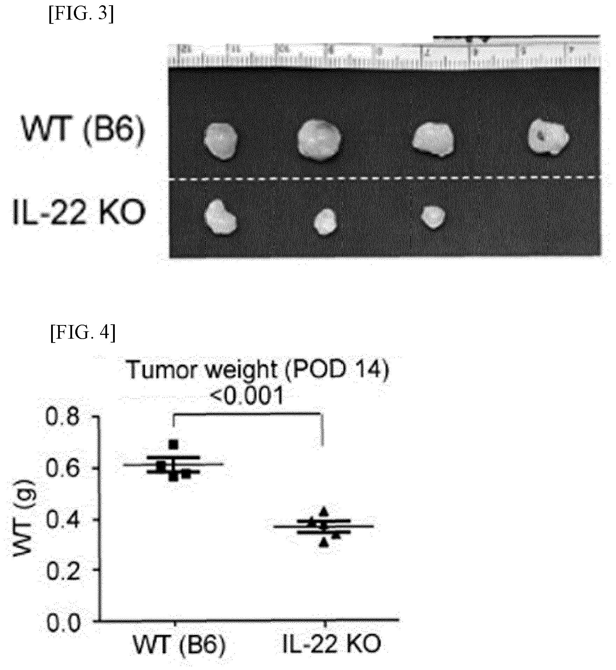

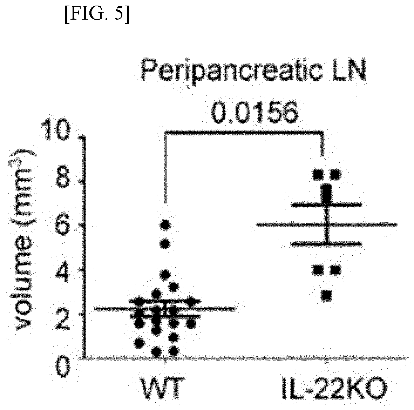

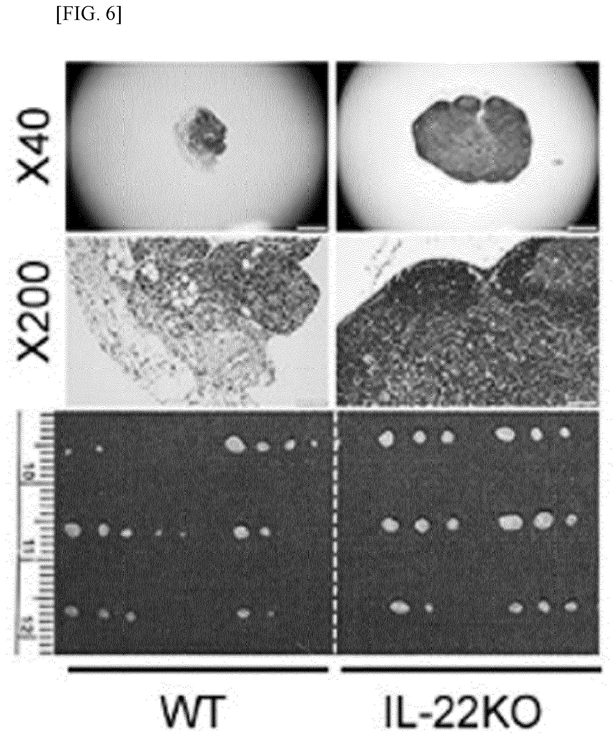

The pancreatic ductal adenocarcinoma cell line (Pan02) was injected directly into the pancreases 8-week old IL-22 gene knockout (K/O) mouse models (B6; 129S5-ll22tm1lex/Mmucd, Genentech) and 8-week old wild-type (WT) mice (C57BL/6, OrientBio) at a concentration of 2 x 10⁶ cells/20 µL. After 14 days, the pancreatic tumors were photographed and the results are shown in FIG. 3. Also, the weights of the pancreatic tumors were measured and the results are shown in FIG. 4. In addition, the volumes of the peripancreatic lymph nodes are measured and the results are in FIG. 5. Furthermore, photographs showing microscopic observation of the pancreatic tumor tissues are shown in FIG 6.

As shown in FIGS. 3 and 4, it could be confirmed that the size of the pancreatic tumor in the IL-22 gene knockout mouse models injected with the pancreatic cancer cells decreased compared to that in the wild-type mice.

As shown in FIGS. 5 and 6, it could be seen that, in the IL-22 gene knockout mouse models, the lymph nodes were activated, but in the wild-type mice, the lymph nodes were atrophied and the tumor cells infiltrated the lymph nodes.

### [Experimental Example 31

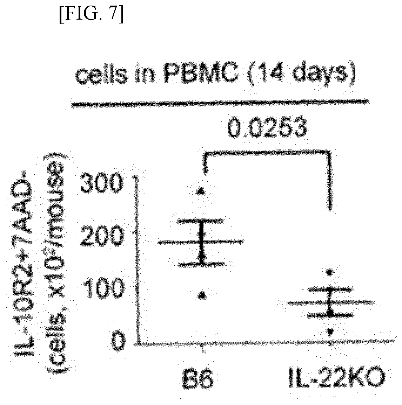

An experiment was conducted in the same manner as in Experimental Example 2. On day 14 after injection of the pancreatic cancer cells, PBMCs were collected from the mice, and IL-10R2+7AAD- cells, IL-10R2+CD11b+ cells and IL-10R2+CD11b+7AAD- cells were separated using a flow cytometer and the number of the cells was measured. The results of the measurement are shown in FIGS. 7 to 9, respectively.

As shown in FIG. 7, it could be confirmed that the number of IL-10R2+7AAD- cells in the IL-22 gene knock mouse models significantly decreased compared to that in the wild-type mice.

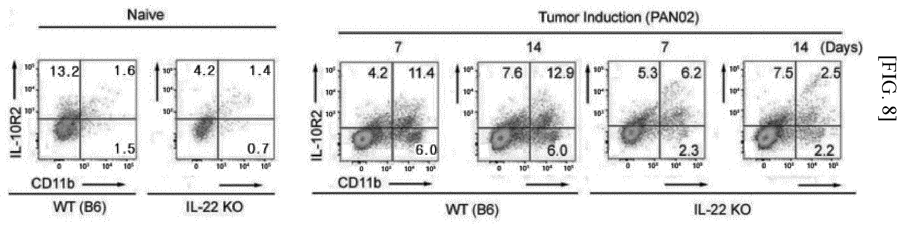

As shown in FIG. 8, it could be confirmed that the number of IL-10R2+CD11b+ cells increased in the mice injected with the pancreatic cancer cells, but the number of IL-10R2+CD11b+ cells decreased in the IL-22 gene knockout mouse models compared to the wild-type mice.

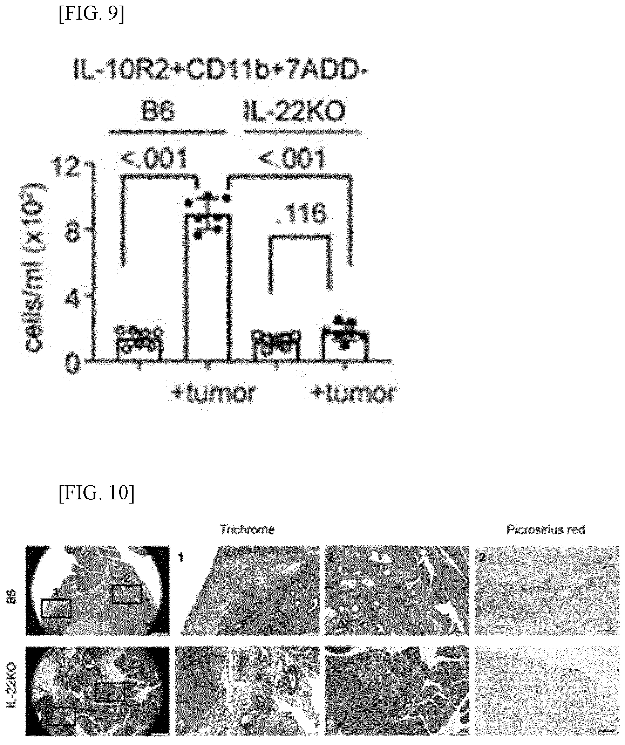

As shown in FIG. 9, it could be confirmed that the number of IL-10R2+CD11b+7AAD- cells significantly increased in the mice injected with the pancreatic cancer cells, but the number of IL-10R2+CD11b+7AAD- cells in the IL-22 gene knockout mouse models decreased to that in the mice not injected with the pancreatic cancer cells, compared to the wild-type mice.

### [Experimental Example 41

An experiment was conducted in the same manner as in Experimental Example 2. On day 14 after injection of the pancreatic cancer cells, the mice were euthanized, and the pancreas tissue was taken from each mouse, stained with trichrome and Picrosirius red, and then observed with a microscope. Photographs of the observation are shown in FIG. 10.

As shown in FIG. 10, it could be confirmed that the wild-type mice (B6) injected with the pancreatic cancer cells were much thicker and had more fibrosis than the IL-22 gene knockout mouse models (IL-22KO).

### [Experimental Example 51

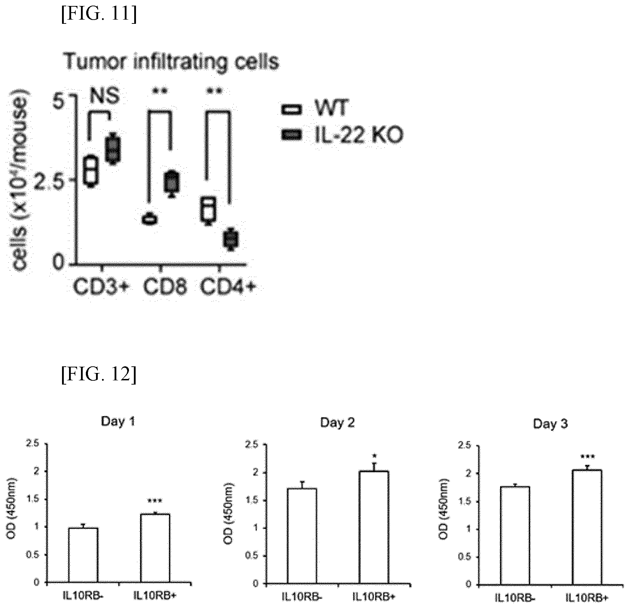

An experiment was conducted in the same manner as in Experimental Example 2. On day 14 after injection of the pancreatic cancer cells, the tumor-infiltrating cells, CD3+ cells, CD8+ cells, and CD4+ cells, were separated using a flow cytometer, and the number of the cells was measured. The results of the measurement are shown in FIG. 11.

As shown in FIG. 11, it could be confirmed that the number of CD8+ cells in the IL-22 gene knockout mouse models increased compared to that in the wild-type mice, and the number of CD4+ cells decreased in the IL-22 gene knockout mouse models. Thereby, it could be seen that, in the IL-22 gene knockout mouse models, cancer immune evasion was restored and cytotoxic immunity corresponding to the cancer cells was restored.

### [Experimental Example 61

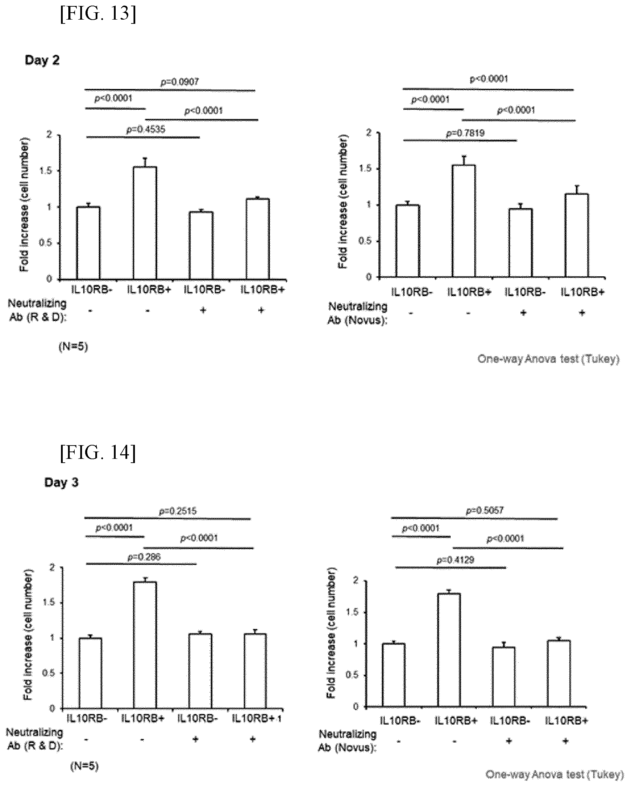

PanO2 cells were dispensed into a 96-well plate at a density of 5 x 10³ cells(100 µl/well) (n=5), and then pre-cultured in a humidified incubator at 37°C under 5% CO₂. The PanO2 cells were cultured for 24 hours, 48 hours or 72 hours in 200 µl of conditioned medium (IL-10RB+, IL-10RB-) supplemented with 2 µl/ml of an anti-IL-10R2 neutralizing antibody (R&D Systems, Catalog number: MAB874) or 1 µl/ml of an anti-IL-10R2 neutralizing antibody (Novus Biologicals, Catalog number: NBP211654), which binds to the epitope represented by SEQ ID NO: 7. 10 µl of CCK-8 solution was added to each well of the plate, and then each well was incubated in an incubator for 3 hours. The absorbance at 450 nm was measured using a microplate reader, and the results are shown in FIG. 12. In addition, number of cells based on the IL-10RB- conditioned medium was measured, and the results are shown in FIGS. 13 and 14.

As shown in FIG. 12, it could be confirmed that the number of the pancreatic cancer cells further increased in the IL-10RB+ conditioned medium compared to the IL-10RB- conditioned medium.

As shown in FIGS. 13 and 14, it could be confirmed that treatment with the anti-IL-10R2 antibody significantly decreased the number of the pancreatic cancer cells.

### [Experimental Example 71

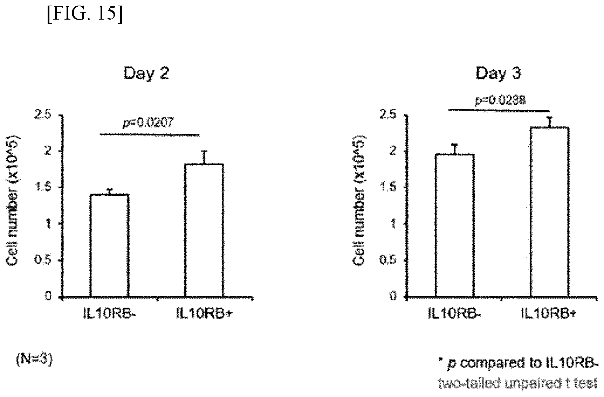

3.0 x 10⁶ Pano2 cells were labeled with CellTracker^{™} Green CMFDA (5-chloromethylfluorescein diacetate), and then the Pano2 cells were cultured at a density of 1 × 10⁵ cells per well (n=3). Thereafter, 1 × 10⁵ IL-10R2+ cells or IL-10R2- cells were added to each well, and then cultured for 48 hours or 72 hours. The cell number of the Pano2 cells was measured using a hemocytometer, and the results of the measurement are shown in FIG. 15.

As shown in FIG. 15, it could be confirmed that the number of the pancreatic cancer cells further increased when the IL-10R2+ cells were added compared to when the IL-10R2- cells were added.

### [Experimental Example 81

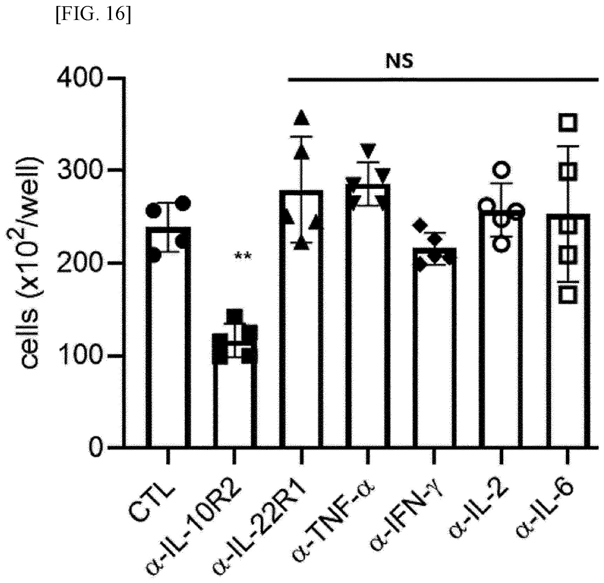

3.0 x 10⁶ Pano2 cells were labeled with CellTracker^{™} Green CMFDA (5-chloromethylfluorescein diacetate), and then the Pano2 cells were cultured at a density of 1 × 10⁵ cells per well (n=3). Thereafter, the cells were treated with neutralizing antibodies specific to IL-10R2, IL-22R1, TNF-α, IFN-γ, IL-2 and IL-6, and cultured for 48 hours. The cell number of the Pano2 cells was measured using a hemocytometer, and the results of the measurement are shown in FIG. 16.

As shown in FIG. 16, it could be confirmed that the number of the pancreatic cancer cells significantly decreased only when the cells were treated with the anti-IL-10R2 antibody.

### [Experimental Example 91

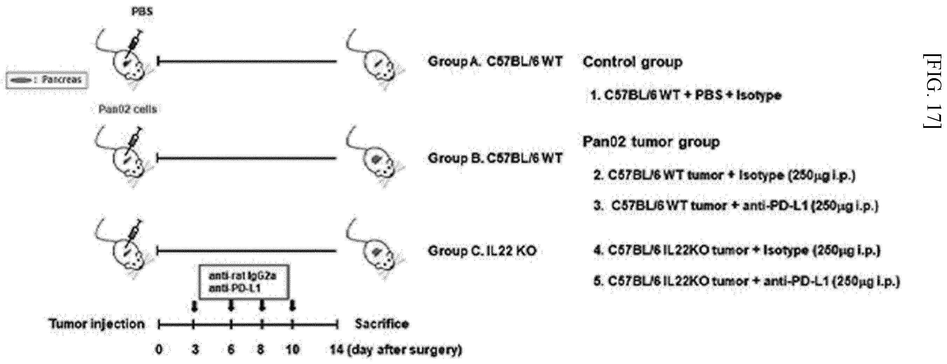

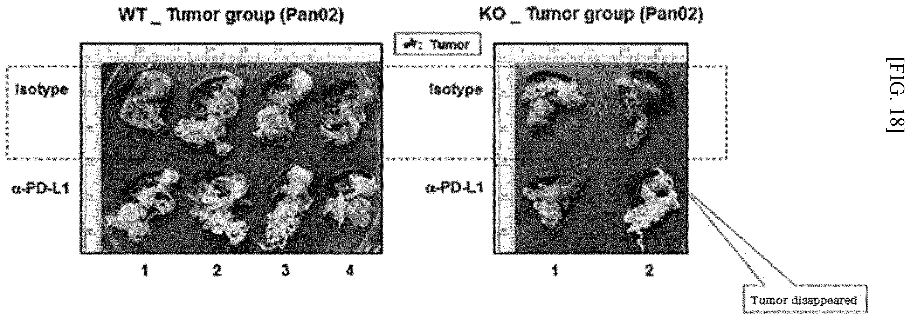

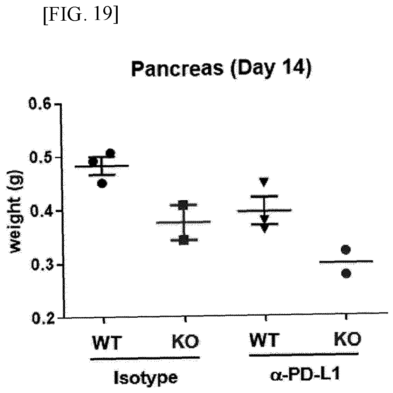

FIG. 17 schematically shows a design of the following experiment. 2 x 10⁶ cells/20 µL of Pan02 PDACs (pancreatic ductal adenocarcinoma cell line (Pan02) were injected directly into the pancreases of 8-week-old IL-22 gene knockout (K/O) mouse models (B6; 129S5-ll22tm1lex/Mmucd, Genentech) and 8-week-old wild-type (WT) mice (C57BL/6, OrientBio). Days 3, 6, 8, and 10 after surgery, each of an anti-PD-Ll antibody (250 µg i.p.) and an anti-mouse IgG2 antibody (250 µg i.p.) (isotype: a negative control for PD-L1 antibody) as an isotype antibody was injected into the wild-type mice and IL-22 K/O mouse group to which the Pan02 cells have been administered. As a control, PBS and an anti-mouse IgG2 antibody (250 µg i.p.) were injected into 8-week-old wild-type (WT) mice (C57BL/6, OrientBio) on days 3, 6, 8 and 10. On day 14, the mice were sacrificed, the pancreatic tumors were photographed, and the results are shown in FIG. 18. In addition, the weight of the pancreas including the tumor for each group was measured, and the results are shown in FIG. 19.

As shown in FIG. 18, it could be confirmed that, when both IL-22 gene knockout according to the present invention and administration of the anti-PD-Ll antibody were performed, the tumor suppression effect was better than when only IL-22 gene knockout or only administration of the anti-PD-L1 antibody was performed, and thus the tumor disappeared.

In addition, as shown in in FIG. 19, it could be confirmed that the weight of the pancreas including the tumor significantly decreased when both IL-22 gene knockout and administration of the anti-PD-Ll antibody were performed compared to when only IL-22 gene knockout was performed.

### [Experimental Example 101

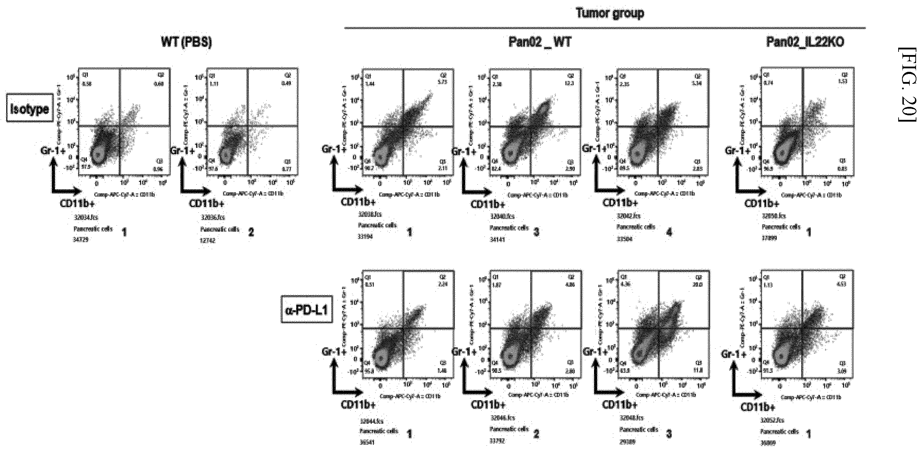

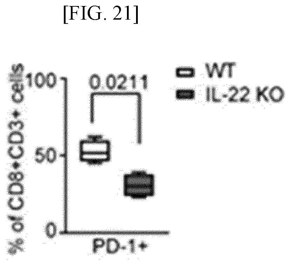

The immune cells infiltrating into the pancreas tissue isolated for each group in Experimental Example 9 above were separated, stained with the cytotoxic T cell markers CD3 antibody and CD8 antibody, and subjected to FACS analysis. The results of the analysis are shown in FIGS. 20 and 21.

As shown in FIGS. 20 and 21, it could be confirmed that, when both IL-22 gene knockout according to the present invention and administration of the anti-PD-Ll antibody were performed, the number of CD3+ CD8+ cells significantly decreased compared to when only IL-22 gene knockout was performed.

Although the embodiments of the present invention have been described in detail above, the scope of the present invention is not limited thereto, and it will be apparent to one of ordinary skill in the art that various modifications and variations are possible, without departing from the technical spirit of the present invention as defined in the appended claims.

### Industrial Applicability

The present invention is intended to provide a composition capable of preventing or treating cancer, particularly pancreatic cancer.

## Claims

1. A pharmaceutical composition for preventing or treating cancer containing, as an active ingredient: an inhibitor of activity of at least one of interleukin-10 receptor subunit beta (IL-10R2, IL-10RB) and interleukin-22 (IL-22); or an inhibitor of expression of a gene encoding at least one of the interleukin-10 receptor subunit beta and the interleukin-22.

2. The pharmaceutical composition of claim 1, further containing an inhibitor of activity of programmed death ligand 1 (PD-L1) or an inhibitor of expression of a gene encoding the programmed death ligand 1.

3. The pharmaceutical composition of claim 1, wherein the interleukin-10 receptor subunit beta or the interleukin-22 is present in CD45⁺ cells.

4. The pharmaceutical composition of claim 1, wherein the inhibitor of the activity comprises any one or more selected from the group consisting of compounds, peptides, peptide mimetics, aptamers, antibodies, and natural products, which bind specifically to the interleukin-10 receptor subunit beta or the interleukin-22.

5. The pharmaceutical composition of claim 1, wherein the inhibitor of the expression comprises any one or more selected from the group consisting of an antisense nucleotide, small interfering RNA (siRNA), short hairpin RNA (shRNA), and ribozyme, which bind complementarily to the gene encoding the interleukin-10 receptor subunit beta or the interleukin-22.

6. The pharmaceutical composition of claim 1, wherein the inhibitor of the activity binds specifically to an epitope of interleukin-10 receptor subunit beta represented by SEQ ID NO: 7.

7. The pharmaceutical composition of claim 1, wherein the cancer is pancreatic cancer, thyroid cancer, breast cancer, biliary tract cancer, gallbladder cancer, colorectal cancer, uterine cancer, esophageal cancer, gastric cancer, brain cancer, rectal cancer, lung cancer, bladder cancer, kidney cancer, ovarian cancer, prostate cancer, uterine cancer, head and neck cancer, skin cancer, blood cancer or liver cancer.

8. A method for preventing or treating cancer comprising administering, to a subject in need of administration, a pharmaceutical composition containing, as an active ingredient: an inhibitor of activity of at least one of interleukin-10 receptor subunit beta (IL-10RB, IL-10R2) and interleukin-22 (IL-22); or an inhibitor of expression of a gene encoding at least one of the interleukin-10 receptor subunit beta and the interleukin-22.
